(19) Europäisches Patentamt

European Patent Office

Office européen des brevets

(11) **EP 3 870 059 B1**

(12) **EUROPEAN PATENT SPECIFICATION**

(45) Date of publication and mention
of the grant of the patent:
**20.12.2023 Bulletin 2023/51**

(21) Application number: **19783067.2**

(22) Date of filing: **11.10.2019**

(51) International Patent Classification (IPC):
*A61B 8/06* (2006.01)    *G01S 15/89* (2006.01)
*G06T 7/00* (2017.01)    *A61B 8/08* (2006.01)
*G06T 7/62* (2017.01)    *G06T 7/149* (2017.01)
*G06T 7/12* (2017.01)    *G01S 7/52* (2006.01)

(52) Cooperative Patent Classification (CPC):
**G01S 15/8925; A61B 8/06; A61B 8/085;**
**A61B 8/488; A61B 8/5223; G01S 7/52066;**
**G01S 15/8915; G01S 15/8979; G06T 7/12;**
**G06T 7/149; G06T 7/62;** G01S 15/8984;
G06T 2207/10136; G06T 2207/30104

(86) International application number:
**PCT/EP2019/077550**

(87) International publication number:
**WO 2020/083660 (30.04.2020 Gazette 2020/18)**

(54) **METHODS AND SYSTEMS FOR DERIVING A PARAMETER RELATING TO FLOW FROM A BLOOD VESSEL**

VERFAHREN UND SYSTEME ZUR ABLEITUNG EINES PARAMETERS IM ZUSAMMENHANG MIT DEM FLUSS AUS EINEM BLUTGEFÄSS

PROCÉDÉS ET SYSTÈMES PERMETTANT DE DÉRIVER UN PARAMÈTRE RELATIF AU FLUX D'UN VAISSEAU SANGUIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.10.2018  IN 201841039786
13.02.2019  US 201962804845 P
14.03.2019  EP 19162788**

(43) Date of publication of application:
**01.09.2021 Bulletin 2021/35**

(73) Proprietor: **Koninklijke Philips N.V.
5656 AG Eindhoven (NL)**

(72) Inventors:
• **SHULEPOV, Sergei Y.
5656 AE Eindhoven (NL)**
• **DE WILD, Nico Maris Adriaan
5656 AE Eindhoven (NL)**
• **SETHURAMAN, Shriram
5656 AE Eindhoven (NL)**
• **PALANISAMY, Krishnamoorthy
5656 AE Eindhoven (NL)**
• **MATHEW, Denny
5656 AE Eindhoven (NL)**

(74) Representative: **Philips Intellectual Property &
Standards
High Tech Campus 52
5656 AG Eindhoven (NL)**

(56) References cited:
**US-A- 6 071 242        US-A1- 2012 078 106
US-A1- 2014 303 499     US-B1- 6 663 568**

**Description**

FIELD OF THE INVENTION

The invention relates to the field of ultrasound, and more specifically to the field of ultrasonic blood flow measurement.

BACKGROUND OF THE INVENTION

Continuous and accurate blood flow measurements from a blood vessel are essential in hemodynamic monitoring (HDM) applications. It is possible to provide a subject with an ultrasound-based patch that will continuously quantify and monitor blood flow parameters. The subject may, for example, be in emergency and/or peri-operative care.

Full ultrasound imaging and diagnostics of the blood vessels involves a combination of structural imaging, such as B-mode imaging, and Doppler imaging, such as color flow and/or spectral Doppler imaging. In ultrasound diagnostic imaging, Doppler measurements are typically performed by skilled sonographers, wherein the sonographer positions a Doppler imaging plane in the middle of the vessel and aligned with the main axis, which extends along the length of the vessel. Locating such a plane in a vessel by an untrained operator can be cumbersome.

Further, with a body-worn ultrasound patch for continuous subject monitoring, there may be no visual ultrasound feedback (i.e., in the form of an ultrasound image) to the operator. Therefore, the operator may also be placing the sensor in the absence of an ultrasound image of the vessel, thereby increasing the difficulty of the task.

Another consideration for continuous ultrasound monitoring is that the subject may move with the sensor attached during active monitoring. Under these conditions, it may be challenging to place and maintain the position of the ultrasound patch during monitoring so that it is correctly aligned with the vessel axis.

Further, since the vessels are typically relatively small in diameter, any motion causing displacement of the sensor off the vessel axis may dramatically affect the measured vessel diameter as well as any detected blood flow values.

There is therefore a need for a means of acquiring and continually monitoring blood flow in an accurate and robust manner.

Document US 2012/0078106 discloses a method for continuous non-invasive monitoring of multiple arterial parameters of a patient.

Document US 6,663,568 discloses a method of estimating the volume flow in a vessel utilizing ultrasound techniques including the steps of imaging the vessel utilizing an ultrasonic transducer array so as to produce fluid velocity information for the vessel.

SUMMARY OF THE INVENTION

The invention is defined by the claims.

According to examples in accordance with an aspect of the invention, there is provided a method for obtaining a parameter relating to flow from a vessel according to claim 1.

The method provides for the deriving of a parameter relating to flow from a vessel. More specifically, the method provides for a robust means for deriving a parameter relating to flow from a vessel when an angle exists between the imaging plane and the vessel axis, which extends along the length of the vessel.

Typically, data relating to the flow within a vessel is derived using an imaging plane that is aligned with the vessel axis. However, this method is both difficult to apply and is susceptible to motion artifacts.

By establishing an imaging plane that intersects the vessel at a given angle (or within a given range of angles) to the vessel axis and taking account of said angle when deriving the parameter relating to flow, the establishing of the imaging plane is greatly simplified and the measurements are made more robust to motion artifacts.

In an embodiment, deriving the parameter relating to flow comprises determining a flow velocity.

In this way, blood flow velocity may form part of the parameter relating to flow.

In an arrangement the Doppler angle is between 10 and 50 degrees, for example between 15 and 40 degrees.

In an example, fitting the general ellipse equation to the identified vessel cross section is based on a non-linear least squares method.

In this way, the shape of the vessel cross section (which is typically elliptical when the imaging plane intersects the vessel axis at an angle) may be determined in a computationally efficient manner.

In the method according to claim 1, determining the shape of the identified vessel cross section comprises applying an image momentum method to the ultrasound data.

In this way, a shape recognition algorithm may be applied to the ultrasound data in order to accurately establish the shape of the vessel cross section.

In an arrangement, the method further comprises:

Generating, by communicating to the ultrasound transducer (sensor), a beam steering angle based on the vessel axis; and

Adjusting, by communicating to the ultrasound transducer (sensor), the position of the imaging plane based on the beam steering angle.

In this way, automatic adjustments may be made to the location of the imaging plane using electronic beam steering.

In an embodiment, the method further comprises applying a dynamic gating to the Doppler ultrasound data.

By employing a dynamic gating scheme, the gating used to assess the ultrasound data may be adjusted (in both size and position) in order to remain in an optimal position relative to the vessel.

In an arrangement, determining the shape of the identified vessel cross section comprises identifying a vessel diameter.

In a further arrangement, the method further comprises:

monitoring, by a processor, a change in vessel diameter over time; and

determining, by a processor, a measure of vessel distensibility based on the change in vessel diameter over time.

In an embodiment, the parameter relating to flow comprises one or more of:

a flow rate; and

a flow distribution.

In this way, the flow rate and/or flow distribution through the vessel may be determined, thereby providing a measure of the volume of blood flowing through the vessel over time.

In an embodiment, the ultrasound data comprises B-mode data.

According to examples in accordance with an aspect of the invention, there is provided a computer program comprising computer program code means which is adapted, when said computer program is run on a computer, to implement the method as described above.

According to examples in accordance with an aspect of the invention, there is provided a medical system according to claim 11.

In an embodiment, the system further comprises an ultrasound transducer in communication with the processor, wherein the ultrasound transducer is adapted to acquire ultrasound data from an imaging plane.

In a further embodiment, the ultrasound transducer comprises one or more of:

a linear transducer array;

a T-shaped transducer array; and

a 2D transducer array.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows an ultrasound diagnostic imaging system to explain the general operation;

Fig. 2 shows a method of the invention;

Fig. 3a shows a schematic representation of an ultrasound transducer collecting data in a typical manner;

Fig. 3b shows a schematic representation of an ultrasound transducer collecting data according to the method of Fig. 2;

Fig. 4 shows a schematic representation of the relationship between the Doppler angle, between the vessel axis and the imaging plane, and the shape of a detected ellipse;

Fig. 5 shows a Doppler gate positioned at a centroid of an identified elliptical vessel cross section;

Fig. 6 shows an example of determining a vessel diameter;

Fig. 7 shows an example workflow of the guided placement of a sensor comprising a single linear transducer array; and

Fig. 8 shows a graphical representation of an example of guided sensor placement using a T-shaped array and visual indicators disposed on the sensor.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention, as defined by the appended claims.

These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for obtaining a parameter relating to flow from a vessel according to claim 1. The method begins by obtaining ultrasound data, which includes Doppler ultrasound data, from an imaging plane and identifying a vessel cross section within the imaging plane based on the ultrasound data. A shape of the identified vessel cross section is then determined and a vessel axis extending along the length of the vessel is determined based on the shape of the identified vessel cross section, with the assumption of a circular cross section on a plane perpendicular to the vessel axis. A Doppler angle is determined between the vessel axis and the imaging plane and the parameter relating to flow derived based on the Doppler angle, the vessel axis and the Doppler ultrasound data.

As discussed above, traditional ultrasound monitoring workflows using standard scanners are not optimal for prolonged, operator-free monitoring. More specifically, the alignment of the imaging plane of the ultrasound transducer with the vessel axis is difficult to achieve and easily disrupted by motion of the subject. The invention provides for a more robust method of monitoring blood flow parameters by allowing the imaging plane to intersect with the vessel axis at a given angle, which is then corrected for. However, this approach requires a different workflow and different reconstruction algorithms, compared to a traditional vascular ultrasound imaging and diagnostic workflow, which are described below.

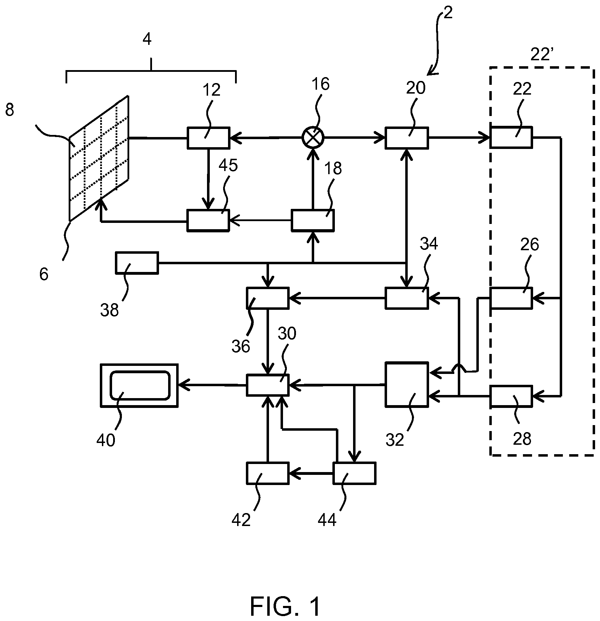

The general operation of an exemplary ultrasound system will first be described, with reference to Fig. 1, and with emphasis on the signal processing function of the system since this invention relates to the processing of the signals measured by the transducer array.

The system comprises an ultrasound array transducer sensor (probe) 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

It shall be understood by the skilled person that the functions of the signal processor 22; B-mode processor 26 and a Doppler processor 28 can be performed by a single processor 22'.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The methods described herein may be performed on a processing unit. Such a processing unit may be located within an ultrasound system, such as the system described above with reference to Fig. 1. For example, the image processor 30 described above may perform some, or all, of the method steps detailed below. Alternatively, the processing unit may be located in any suitable system, such as a monitoring system, that is adapted to receive an input relating to a subject.

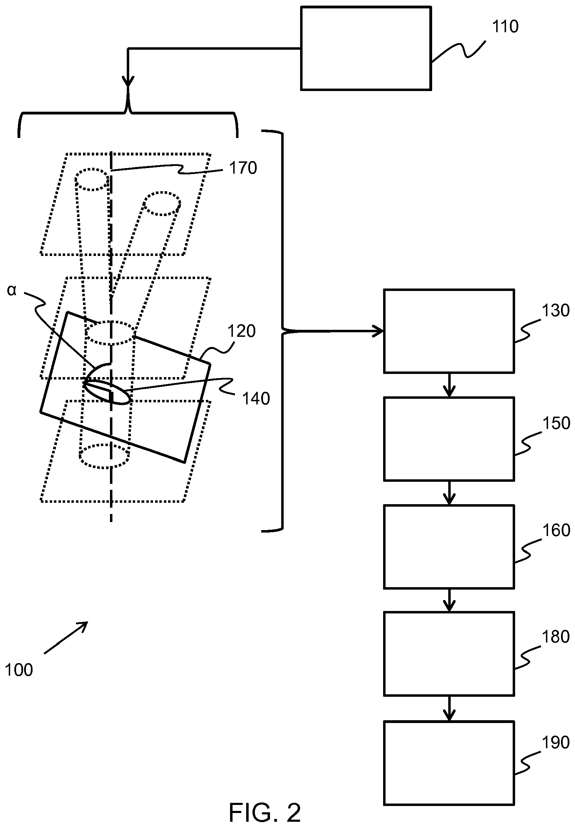

Fig. 2 shows a method 100, which may be executed by the processor 22' for obtaining a parameter relating to flow from a vessel.

The method begins in step 110 by obtaining ultrasound data representative of an imaging plane 120, for example, acquired by the ultrasound sensor 4. The ultrasound data comprises Doppler ultrasound data, depicting motion within the imaging plane such as blood flow. The ultrasound data may also include B-mode data, which depicts the structural features within the imaging plane. The method may be performed using Doppler ultrasound data alone or in combination with B-mode data.

The ultrasound data may obtained from a single imaging plane crossing the vessel at any point.

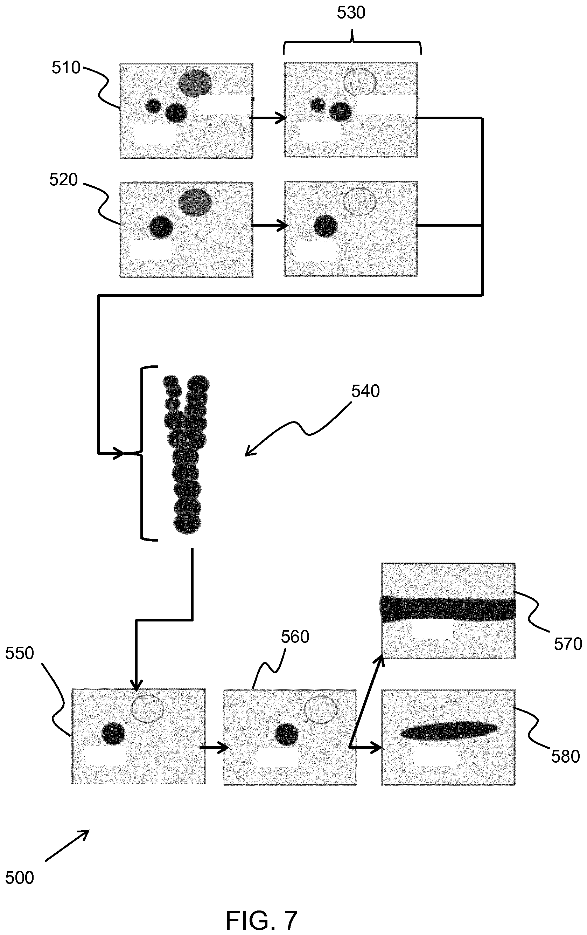

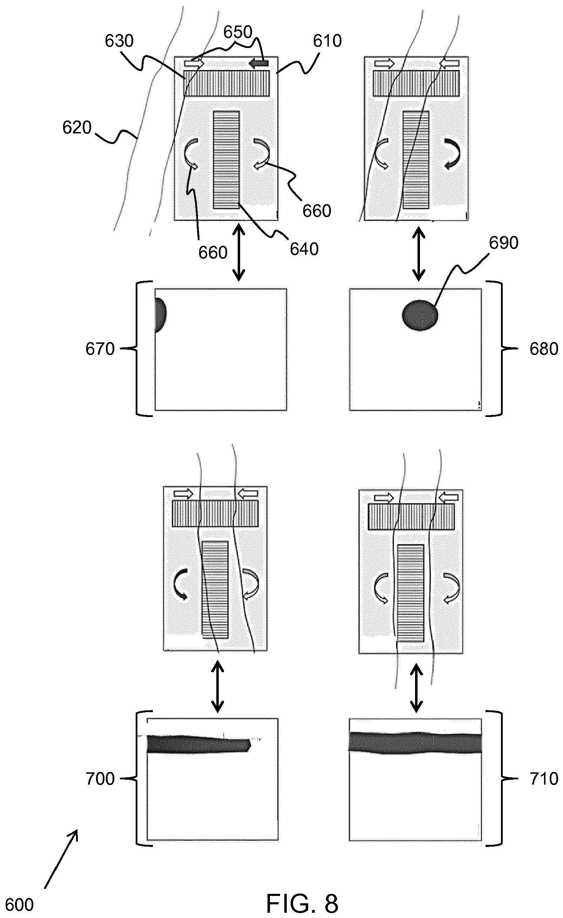

The ultrasound data may be obtained by way of an ultrasound transducer. Further, the placement and positioning of the ultrasound transducer may be guided such that the imaging plane defined by the transducer interests the vessel axis within a given angle. The angle, which may be referred to as a Doppler angle, may be between 10 and 50 degrees, for example between 15 and 40 degrees. Examples of how an ultrasound transducer may be guided to a given position are described below with reference to Figs. 7 and 8.

In this way, the ultrasound transducer (sensor) used to acquire the ultrasound data does not need to be aligned with the vessel at a given specific angle, which may be difficult to achieve in practice. Rather, the sensor may be placed at an angle within the range of 10 to 50 degrees with respect the central axis of the vessel and achieve optimal data acquisition results. In this way, the positioning of a sensor becomes very robust towards patient motion and is not susceptible to the same sensitivity as a traditional ultrasound data acquisition method. Further, the elimination of the requirement to place the sensor in a specific orientation reduces the time and skill required to properly locate the probe for data acquisition.

The ultrasound data may include a variety of data. For example, the ultrasound data may include ultrasound image data, which may then be used to represent the imaging planes as 2D ultrasound images. Further, image segmentation may be employed to recognize and identify vessel cross sections.

Ultrasound image data may include structural B-mode data, in addition to the Doppler ultrasound data, which may not be displayed as an image to a user but may be employed in a process internal to the ultrasound system.

Further, or alternatively, the ultrasound data may include pulsatility data, which represents the variance of the blood flow velocity within a given vessel. In the example of pulsatility data, the vessels may be identified by way of matching the captured pulsatility data to known pulsatility profiles, such as the typical pulsatility profile of a carotid artery or jugular vein.

The combination of ultrasound image data (B-mode data) and Doppler (color) data may be referred to as duplex data-based vessel identification.

In step 130, a vessel cross section 140 is identified within the imaging plane based on the ultrasound data. For example, the vessel cross section may be identified based on the flow characteristics present in the Doppler ultrasound data. This step can be performed by either the Doppler processor 28 forming a part of the processor 22'. Alternatively, the vessel cross section may be identified by analyzing intensity variation of the B-mode data. In this case the B-mode processor 26 forming a part of the processor 22" might perform this identification.

As stated above, the ultrasound data may include a variety of data. For example, the ultrasound data may include ultrasound image data, which may then be used to represent the imaging plane as a 2D ultrasound image. Further, image segmentation may then be employed to recognize and identify the vessel cross section.

Further, the ultrasound data includes Doppler ultrasound data. In this case, the identification of the vessel cross section in the imaging plane may include determining a direction of blood flow relative to the transducer within the imaging plane based on the Doppler ultrasound data.

The direction of blood flow within an imaging plane, and more specifically within a given area of an imaging plane, may be used to identify a vessel. For example, where an imaging plane is located at a neck of a subject, two vessels (the carotid artery and the jugular vein) may be identified by way of their opposing flow directions.

By way of an example, a vessel cross section may be identified according to the following steps. Firstly, the color Doppler flow image is converted to HSV (Hue, Saturation, Value) space and the image is converted to a binary image by applying threshold saturation to HSV space. The binary image is then pre-processed by filling the holes in the image and the region having the largest area within the image is selected by way of connected component analysis.

The center of a vessel is identified from the centroid of the selected region and the RGB patterns of the selected region are analyzed to differentiate the laminar and turbulent flow within the vessel. Indeed, depending on the medical application this information may be used as a guide to maintain a current position or to move away from the bifurcation. A pulsatility measure is then computed from the Doppler color signal to confirm that the signal is from a given vessel, such as an artery.

In step 150, the shape of the identified vessel cross section is determined.

Determining the shape of the identified vessel cross section may be performed by fitting a general ellipse equation to the identified vessel cross section based on a nonlinear least squares method. The shape of the identified vessel cross section is determined by applying an image momentum method to the ultrasound data.

In step 160, the vessel axis 170, which extends along the length of the vessel, is determined based on the shape of the identified vessel cross section. This determination is based on an assumption of a circular cross section on a plane perpendicular to the vessel axis.

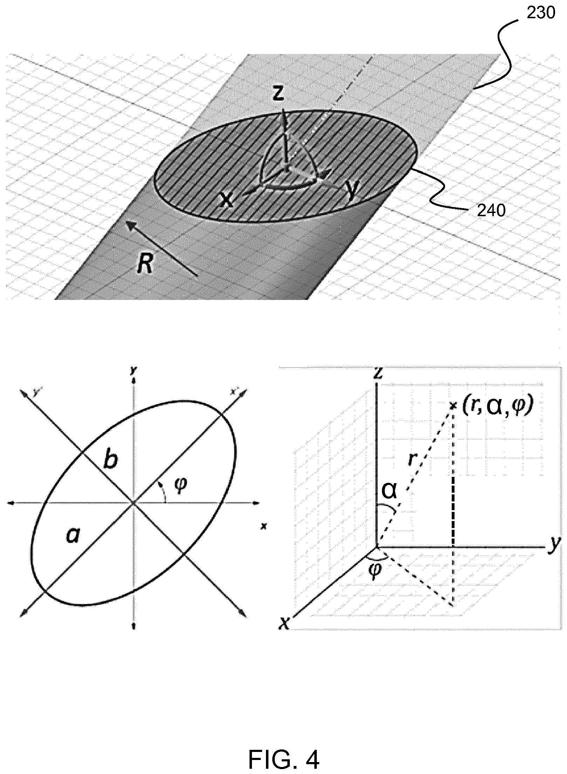

The determining of the shape of the vessel cross section and the vessel axis is discussed below with reference to Fig. 4.

In step 180, a Doppler angle ($\alpha$) between the vessel axis and the imaging plane is determined. The Doppler angle may be between 10 and 50 degrees, for example between 15 and 40 degrees.

In step 190, the parameter relating to flow in the vessel may be derived based on the Doppler angle, the vessel axis and the Doppler ultrasound data.

It should be noted that the method may operate on a continual stream of incoming ultrasound data. Accordingly, the method may be continually repeated in order to update the determined vessel axis and Doppler angle based on the most recently received data. In this way, the method may compensate for subject motion in real time during monitoring. The steps described above may, for example, provide for continuous and accurate blood velocity, or other flow parameter, measurements without any external guidance, providing beat-to-beat output for monitoring.

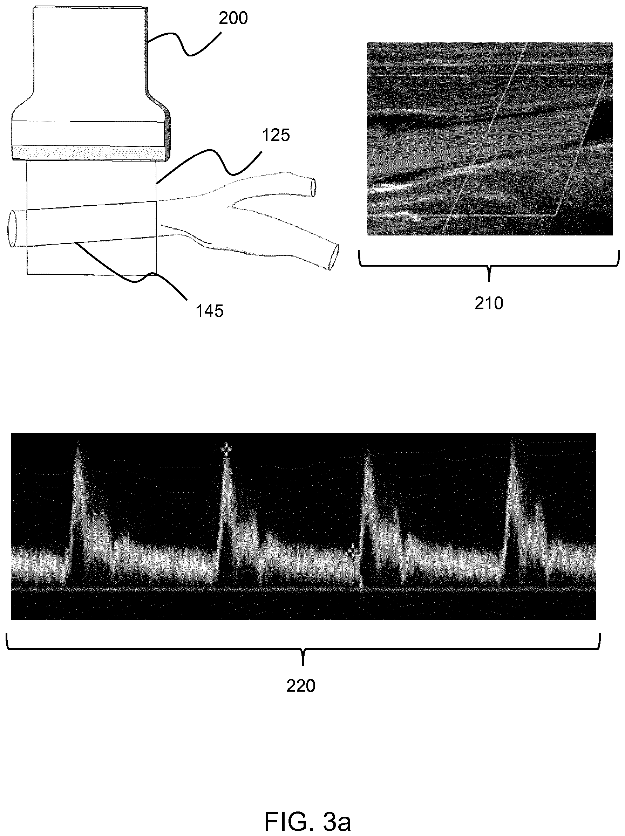

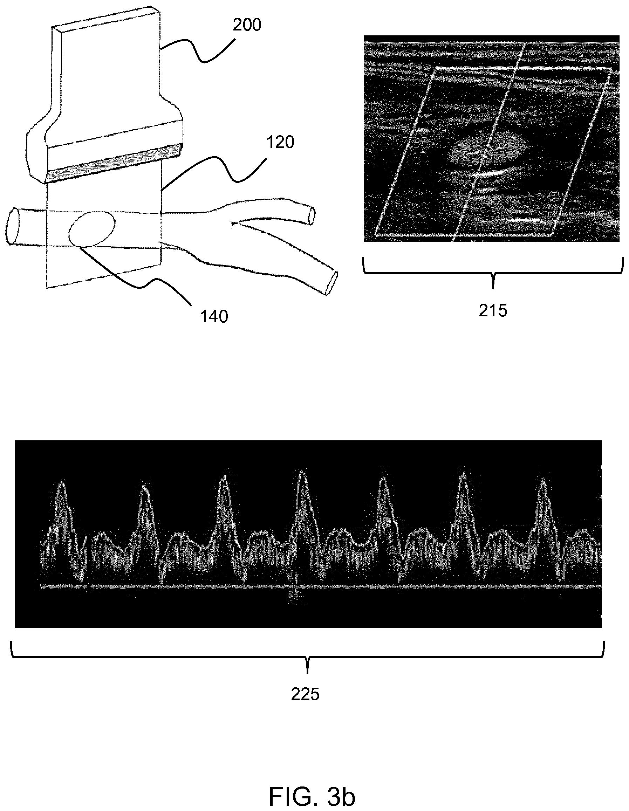

Figs. 3a and 3b show a comparison between capturing ultrasound data from a vessel according to a known method and the method of the invention, respectively.

Fig. 3a shows a schematic representation of an ultrasound transducer 200, such as ultrasound sensor 4, collecting data in a typical manner from an imaging plane 125 aligned parallel with the vessel axis to obtain a rectangular vessel cross section 145. In this case, combined color Doppler and B-mode data 210 is acquired alongside a graph of pulsatility data 220.

The graph of the pulsatility data shows a plot of blood velocity within the vessel (on the y-axis) against time (on the x-axis). As can be seen from the graph, the plot covers approximately four cardiac cycles, wherein each peak represents a constriction of the heart.

Fig. 3b shows a schematic representation of an ultrasound transducer 200 collecting data from an imaging plane 120 according to the method described above with reference to Fig. 2. In this case, combined color Doppler and B-mode data 215 is acquired alongside a graph of pulsatility data 225.

The graph of the pulsatility data shows a plot of blood velocity within the vessel (on the y-axis) against time (on the x-axis). As can be seen from the graph, the plot covers approximately seven cardiac cycles, wherein each peak represents a constriction of the heart.

In addition to the measurement of blood velocity as a parameter relating to flow, following the correction based on the determined vessel axis, within the identified vessel cross section, the vessel diameter is a useful metric to measure.

Fig. 4 shows a schematic representation of the relationship between angle $\alpha$, the Doppler angle between the vessel axis and the imaging plane, and the shape of a detected ellipse.

As described above, the ultrasound transducer may be placed under guidance so that the imaging plane crosses the artery in a desired way. Typically, when a plane crosses an infinite cylinder 230 of radius R at an angle other than 90°, the resulting shape is an ellipse 240. Taking the assumption that a blood vessel may be modeled as an infinite cylinder (i.e. there is no significant change in shape or direction of the vessel within the view of the imaging plane), the vessel cross section will be an ellipse.

When the ellipse is identified, the form-factor (*a* and *b*, dimensions of the principle axes) and its orientation ($\varphi$ and $\alpha$) derived from the color Doppler data stream are sufficient to establish the vessel axis and may be further used to vectorize the flow direction with respect to the imaging plane orientation.

The shape can be fit by a general ellipse equation, using a non-linear least-square method. In this case, the principle Euler angles and the diameter of the vessel will be directly available. Alternatively, shape recognition may be carried out directly in the imaging plane using the image momentum method.

The centroid of the shape may then be used for Doppler gate placement, which is used to measure the parameter relating to flow, and flow vector correction derived from the principal orientation as $1/(\sin\alpha\cdot\cos\varphi)$.

As the parameter relating to flow is being continually derived from a stream of Doppler ultrasound data, it is possible for the flow vector correction to adjust over time in response to external movements, such as the subject moving during examination or monitoring.

The principle angle $\varphi$ is determined by the artery orientation under the skin, while angle $\alpha$ is mainly set by the imaging plane crossing the artery. Therefore, error in the diameter identification can be minimized by optimized sensor placement as discussed above.

There are several approaches that may be employed to determined the shape of a vessel cross section, such as: non-linear shape fitting methods; active shape methods; and image moment methods.

Active shape methods require high quality underlying data and are sensitive to initialization and propagation parameters. In addition, active shape methods are typically computationally expensive.

Non-linear methods may, for example, be based on an iterative (least-square) fitting or direct least square mapping. These methods operate primarily on landmarks and edges of shapes within the ultrasound image.

A non-linear least squares method is a form of least squares analysis used to fit a number of observations ($m$) with a model that is non-linear in a number of unknown parameters ($n$), such that $m \geq n$. The basis of the method is to approximate the model by a linear one and to refine the parameters by successive iterations.

In the example of fitting a general ellipse equation to the vessel cross section, the general ellipse equation acts as the model and is fit to the vessel cross section using, for example, vessel boarders identified identified from Doppler ultrasound data and/or B-mode ultrasound data as the observed data points. The fit may then be iterated a number of times unitl the best fit of the general ellipse equation to the true vessel cross section is found.

Methods of image moments, or image invariants, are based on fitting the planar object using pre-defined primitives. Such methods rely on global planar shape detection and are more robust and computationally effective than other approaches. Image moment methods may also provide for detecting, structuring and tracking multiple objects simultaneously, without significantly increasing computational complexity or demand.

By way of example, an image moment method may progress as follows. Image moments are defined as weighted averages of the image pixel intensities. In a greyscale image with pixel intensities $I(i,j)$, the raw image moment $M(p,q)$ is given by:

$$M_{p,q} = \sum_i \sum_j i^p j^q I(i,j)$$

The image moments of a greyscale image heavily depend on the acquisition settings that the image is acquired with, meaning results of the shape mapping may be sensitive to these settings.

An additional image processing step is performed to yield a binary mask of the image, optimized for a set of imaging parameters. In this way, the method in only required to hande binary images, i.e. image objects consisting of ones and background regions consisting of zeros. For a binary image, the the image moment M($p,q$) for pixels (i,j) that belong to an object (Obj) becomes:

$$M_{p,q} = \sum_{i,j \in Obj} i^p j^q$$

The moments of the image have a direct meaning in relation to the properties of the image. The zeroth-order moment $M_{0,0}$ yields the total number of pixels in the object, i.e. the object area. The first-order moments $M_{1,0}$ and $M_{0,1}$ normalized by $M_{0,0}$, give the coordinates of the barycenter ($\bar{x}, \bar{y}$) in the horizontal ($x$) and vertical ($y$) directions, respectively:

$$\bar{x} = \frac{M_{1,0}}{M_{0,0}} \quad \text{and} \quad \bar{y} = \frac{M_{0,1}}{M_{0,0}}$$

Parameters of the ellipse shape are based on the second-order moments $M_{2,0}$, $M_{1,1}$, and $M_{0,2}$. In order to extract these parameters, the second-order central moments ($\mu'_{i,j}$) have to be constructed first:

$$\mu'_{2,0} = \frac{M_{2,0}}{M_{0,0}} - \bar{x}^2 \quad \mu'_{1,1} = \frac{M_{1,1}}{M_{0,0}} - \bar{x}\bar{y} \quad \mu'_{0,2} = \frac{M_{0,2}}{M_{0,0}} - \bar{y}^2$$

The covariance matrix of the binary object is then given by:

$$cov(Obj) = \begin{bmatrix} \mu'_{2,0} & \mu'_{1,1} \\ \mu'_{1,1} & \mu'_{0,2} \end{bmatrix}$$

The eigenvectors of the covariance matrix above correspond to the major and minor axes of the equivalent ellipse to be fitted to the cross section of the blood vessel. The main region properties of the object (the major and minor axis $l$ and w, and the major axis orientation $\theta$) may then be written as:

$$\theta = \frac{1}{2} \cdot tan^{-1} \left( \frac{2\mu'_{1,1}}{\mu'_{2,0} - \mu'_{0,2}} \right)$$

$$l = \sqrt{8 \left( \mu'_{2,0} + \mu'_{0,2} + \sqrt{4\mu'^2_{1,1} + (\mu'_{2,0} - \mu'_{0,2})^2} \right)}$$

$$w = \sqrt{8 \left( \mu'_{2,0} + \mu'_{0,2} - \sqrt{4\mu'^2_{1,1} + (\mu'_{2,0} - \mu'_{0,2})^2} \right)}$$

Essentially linear operations are carried out on the objects, i.e., operations whichh may easily be vectorized in the sense of computations, and parallelized for multiple region detection, structuring, and tracking.

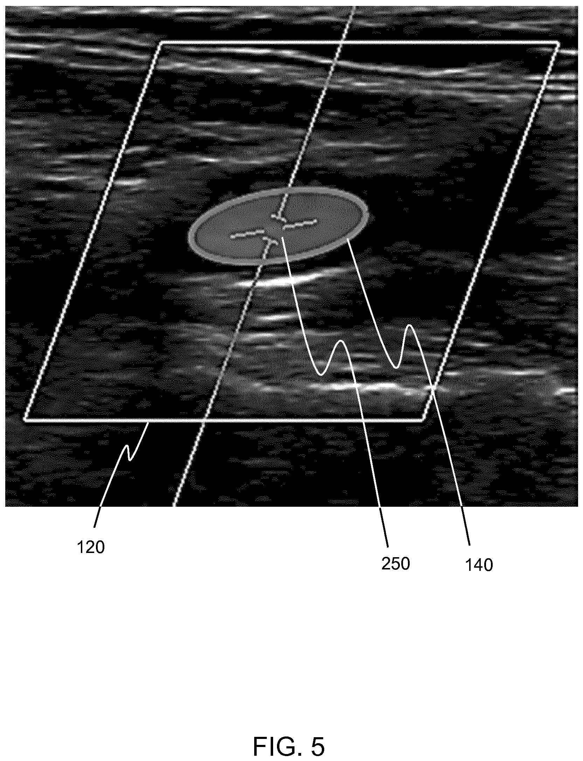

Fig. 5 shows a Doppler gate 250 positioned at a centroid of an identified elliptical vessel cross section 140 within an imaging plane 120.

Information on the vessel orientation with respect to the imaging plane may be determined as described above and used to steer the ultrasound beam to an optimal angle for accurate (pulsed wave) Doppler measurement. However, as the imaging plane is crossing the vessel, static Doppler gating may not perform in an optimal manner.

Therefore, a dynamic Doppler gating concept may be employed in the algorithm. This may be realized in a number of ways. For example, a constant gating size may be used in combination with dynamic positioning of the gate over the centroid of the ellipse identified in the color Doppler data.

Alternatively, a dynamic gating size may be used, which follows the ellipsoidal shape of the color Doppler data. It should be noted that this may include a rectangular shape inscribed into an ellipse, wherein the rectangle has the same aspect ratio as said ellipse. In this case, the system selects the pulsed-wave mode gate, or sample volume size, dynamically to cover the entire vessel under evaluation.

In a further example, multiple gate positions may be used to follow the color Doppler shape, and more specifically the centroid, motion during the cardiac cycle.

The use of dynamic gating (position and/or size) may allow for: a lower mechanical index/thermal index (MI/TI) during continuous monitoring; a better rejection of artifacts during patient movement; and a more accurate reporting of the wave-form.

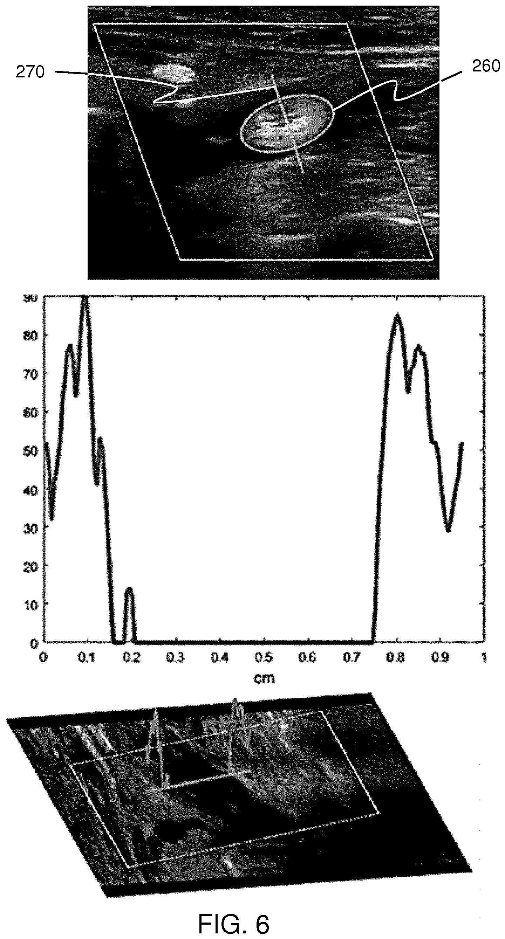

Fig. 6 shows an example of determining a vessel diameter. In the following example, the vessel diameter identification is performed with a combination of color Doppler and B-mode imaging.

Firstly, an active flow area 260 of a vessel, for example a common carotid artery, in the field of view of a sensor is identified using color Doppler data. The active flow area may be identified as described above. The color of the Doppler data indicates the direction of flow relative to the sensor.

The principal directions of the artery are determined from the ellipse shape of color Doppler data crossing between the imaging plane and the vessel axis as described above. Using the principal direction perpendicular to the artery, referred to as a minor axis 270, a 1.5 D segmentation is performed along this direction based on the B-mode pixel (or voxel in the case of 3D ultrasound data) intensity values.

In order to perform such a segmentation, adapted algorithms based on a Chan-Vese-like active contour/snake concept (as described in: Chan, T.F., & Sandberg Y. B(2000). Active contours without edges for Vector-valued Image.

Journal of Visual Communication and Image Representation 11, 130-141 (2000); Chan, T. F., & Vese, L. A. (2001). Active contours without edges. IEEE Transactions on Image Processing, 10(2), 266-277; and Chan, T. F., & Vese, L. A. (2002). A Multiphase level set framework for image segmentation using the Mumford and Shah model. International Journal of Computer Vision 50(3), 271-293, (2002)), and a normalized energy gradient method may be used. The latter is similar to a family of non-linear hybrid detection algorithms (such as, a combination of Sobel and Canny operators as described in Sobel, I., Feldman, G., "A 3x3 Isotropic Gradient Operator for Image Processing", presented at the Stanford Artificial Intelligence Project (SAIL) in 1968 and Canny, J., A Computational Approach To Edge Detection, IEEE Trans. Pattern Analysis and Machine Intelligence, 8(6):679-698, 1986, respectively) using optimized kernels for a given application. These methods are specifically tuned towards imaging performance of the sensor. In order to improve accuracy and robustness, a restricted region of interest (from which the data is obtained) about the principle direction may be specified and the same procedure carried out on a localized 2D area.

Using direct data stream/image processing means that the waveform measurement over the time is not directly connected to one location. This makes parameter extraction more robust against motion of the subject with respect to the monitoring sensor.

This approach may be performed or automatic tracing of the vessel diameter variations due to pulsatile flow in real time. The accuracy of the outcome will depend on the quality of B-mode data. Further, applying this methodology for multiple crossing planes will increase reliability of the algorithms.

In addition, estimating the variation in the vessel diameter across different time instants provides a measure of the distensibility of an artery and can be used to derive additional measurements, such as pressure variation.

In summary, the algorithms presented above are suitable for real-time monitoring of full beat-to-beat waveforms representing blood velocity and flow within a vessel. Together with the real-time segmentation discussed above, the method provides for deriving a parameter relating to flow through the blood vessels using an imaging plane, which is inclined with respect to the vessel axis. Monitoring the real-time diameter of one cardiac cycle will allow for assessing vessel distensibility and associated arterial parameters.

The determined diameter of the vessel may, for example, be combined with derived velocity data in order to determine a flow rate within the vessel. In other words, the vessel diameter and blood velocity may be combined to give a measure of the volume of blood passing through a vessel over time. Further, the parameter relating to flow, which may be flow velocity in this example, may be combined with the vessel diameter in order to identify a flow distribution over the vessel cross section.

As discussed above, the ultrasound data is acquired by way of a sensor. There are a number of implementations of a sensor that may be used to collect the ultrasound data as described. For example, the sensor may comprise: a linear transducer array; two linear transducer arrays, arranged such that the imaging plane of one array is orthogonal to the other; or a 2D array.

Further, the user may be guided as to the placement of the sensor. The guidance provided to the user may be provided in an indirect manner. In other words, the system may analyze the ultrasound data and generate a guidance signal by way of a guidance means separate from the sensor. For example, where a visual guidance signal is generated, an arrow may be displayed on a screen indicating a direction in which the user should move the sensor.

Alternatively, the means for providing guidance to the user may be included in the sensor itself. For example, the sensor may be adapted to generate one or more of: an audible instruction; a visual instruction; an electronic control signal; and a tactile instruction, to guide the user.

Fig. 7 shows an example workflow 500 of the guided placement of a sensor comprising a single linear transducer array, which may be in the form of an ultrasound patch.

Initially, ultrasound data is acquired from a plurality of imaging planes using the ultrasound patch. In the example shown in Fig. 7, a first imaging plane 510 comprises three vessels, shown by the three circles, and a second imaging plane 520 comprises two vessels, shown by the two circles.

Vessel identification 530 is then performed on the imaging planes. The vessel identification may be performed as described above. In the example shown in Fig. 7, the vessel of interest is the carotid artery, which is indicated by the black circles, and the remaining vessel, which in this case is the jugular vein, is disregarded. It may be determined through the color Doppler data and/or the pulsatility data that the two arteries in the first imaging plane and the one artery in the second imaging plane form parts of a common vessel. In other words, it may be determined that a vessel bifurcation exists between the first and second imaging planes.

A sweep 540 may then be performed by moving the sensor along the vessel, thereby mapping out the vessel and the bifurcation in more detail.

The ultrasound patch is then applied by a user with guidance provided by indicators. As stated, indicators may be provided on the patch, as well as, or instead of, being displayed on a remote monitor. The guidance may be based on duplex ultrasound data i.e. a combination of the B-mode data and the color Doppler data. Using the duplex data, the workflow steps also make sure that the patch is placed well below the bifurcation, such that only one vessel cross section of interest exists in the imaging plane as shown in plane 550. For example, the patch may be placed 2 cm below the bifurcation. This ensures that the effects of turbulent flow near the bifurcation are minimized when estimating flow parameters.

The vessel of interest is then centered in the imaging plane as shown in plane 560. In a conventional imaging work flow, the patch is rotated such that a view along the length of the vessel is available, rather than a cross section. The length view may be a complete view 570 or a partial view 580, which may then be corrected through placement guidance or through image correction. Alternatively, the partial view 580 may be sufficient for the intended purpose, meaning that this view may act as the final position of the linear array.

However, according to the methods described above, the imaging plane need not be rotated in line with the vessel axis. Rather, the imaging plane may be maintained at an angle between 10 and 50 degrees, with respect to the vessel axis, in order to derive the parameter relating to flow from the vessel. In other words, the user may be guided to place the sensor in such a position. In this way, the placement of the sensor is simplified and the monitoring is made more robust against interruption due to movement of the subject.

In the case of visual feedback, the sensor may be provided with one or more LEDs, which may provide a visual signal to a user as to how the sensor should be moved. In the case of an audible instruction, one or more speakers may be provided to supply an audible instruction to the user. Where tactile feedback is used, the patch may be provided with one or more vibration modules, which (de)activate to provide a tactile instruction that may be interpreted by the user. In the example of an electronic instruction signal, the feedback may be provided to a remote digital means, such as a monitor, which then presents the user with an instruction in a suitable form.

Fig. 8 shows a graphical representation 600 of an example of guided sensor placement using a T-shaped array and visual indicators disposed on the sensor.

A sensor 610 is shown placed in close proximity to a vessel of interest 620. The sensor comprises a first transducer array 630, which in this case generates a cross sectional view of the vessel, and a second transducer array 640 arranged orthogonally to the first transducer array. The sensor patch may also include further transducer arrays.

Further, the sensor comprises translation visual indicators 650 and rotational visual indicators 660. In operation, a translation visual indicator may be illuminated to provide a user with a guidance signal to move the patch in the indicated direction. Similarly, a rotational visual indicator may be illuminated to provide a user with a guidance signal to rotate the patch in the indicated direction.

In the example shown in Fig. 8, the sensor is placed such that the first transducer array captures an imaging plane that includes an incomplete view 670 of the vessel of interest. The sensor may be initialized by way of a suitable user input, for example a button located on the sensor patch.

Only the first transducer array may be activated at first in order to secure a complete cross sectional view of the vessel of interest. Alternatively, both the first and second transducer arrays may be activated simultaneously.

In this case, the ultrasound sensor patch captures duplex (B-mode and color Doppler) data, which may be streamed to a processor of a connected ultrasound system.

A segmentation algorithm, as described above, is employed to detect the vessels within the imaging plane on the color Doppler data (for example, by searching for the circular appearance of the vessels). If the vessel appears as two segmented regions then the patch is above the bifurcation. The goal in the example shown in Fig. 8 is to place the patch below the bifurcation on the common carotid artery which is a single vessel. An indicator communicates a successful completion of this step if the common carotid artery is detected. As discussed above, the segmentation can be on the color and/or the B-Mode data.

In this instance, the initial view of the vessel is incomplete. Accordingly, a translation visual indicator may be activated on the sensor patch, thereby guiding the used to move the patch to a complete view of the vessel 680.

To ensure that the segmented region 690 is indeed the desired vessel, such as an artery and not a vein or a noise artifact, the sensor may also capture pulsatility data. A pulsatile flow may be used to confirm that the signal is from the desired vessel.

When a complete cross section of the desired vessel is captured a control switch may activate the second transducer array 640 to begin streaming duplex ultrasound data from along the length of the vessel.

An algorithm looks for a cylindrical appearance of the flow spanning the entire imaging plane. If the segmented vessel does not span the lateral of the imaging plane, there may be an angular misalignment of the sensor patch along the length of the vessel 700. The appropriate rotational visual indicators may be used to guide the user to rotate the patch and maximize the cylindrical appearance of the vessel in the ultrasound data. It should be noted that the data need not be visible to the user, but may be performed within the system by the algorithm. Once the desired view 710 is achieved, the indicators may signal the achievement of correct angular alignment. The desired view 710 may change according to the given application. For example, a view of the vessel that results in a long ellipse, rather than a full cylinder may be sufficient. Accordingly, the desired view may include a range of views of the vessel.

Switching back to the cross sectional view of the first transducer array, the algorithm may check if the vessel is still in the center of the view. A positive confirmation indicates that the elements of the second transducer array are aligned with the center axis of the vessel. Otherwise, the algorithm may instruct the user with appropriate indicators to move the sensor patch to achieve that. The user may then activate a button to signal the completion of the patch placement and the various imaging methods described above may proceed.

Once again, according to the methods described above, the imaging plane need not be aligned parallel with the vessel axis. Rather, the imaging plane may be maintained at an angle between 10 and 50 degrees, with respect to the vessel axis, in order to derive the parameter relating to flow from the vessel. In other words, the user may be guided to place the sensor in such a position. In this way, the placement of the sensor is simplified and the monitoring is made more robust against interruption due to movement of the subject.

The ultrasound sensor patch may be a wearable patch that may be temporarily fixed to a subject, thereby allowing them to move freely while the patch is in operation.

In another example, the patch may include a 2D array of transducer elements capable of performing 3D ultrasound imaging. In this case, an x-plane could be used to help in vessel alignment in an analogous way to the first transducer array described above. An x-plane is suggested, rather than using the full 2D array, as this reduces the number of channels required to align the patch, which reduces the power consumption and data rate, and/or allows for higher frame rates. In this case, the patch may simply be placed on an area of interest without requiring iterative movement or adjustment of the patch. All elements of the array may be activated to find the sub-set of elements that best aligns with the vessel axis. There would be no (or minimal) training or expertise required for patch placement in such an example.

In a further example, the linear transducer arrays of the sensor patch described in Fig. 8 may be replaced with 1.5D transducer elements, which would increase the field of view of the imaging plane and may minimize the iterations needed to search for the vessel and/or to align the sensor patch to the vessel.

**Claims**

1. A method (100) for obtaining a parameter relating to flow in a vessel, the method comprising:

   obtaining (110) ultrasound data from an imaging plane (120), wherein the ultrasound data comprises Doppler ultrasound data;
   identifying (130) a vessel cross section (140) within the imaging plane based on the ultrasound data;
   determining (150) a shape of the identified vessel cross section;
   determining (160) a vessel axis (170) based on the shape of the identified vessel cross section based on an assumption of circular cross section on a plane perpendicular to the vessel axis, wherein the vessel axis extends along the length of the vessel;
   determining (180) a Doppler angle ($\alpha$) between the vessel axis and the imaging plane; and
   deriving (190) the parameter relating to flow based on the Doppler angle, the vessel axis and the Doppler ultrasound data, and wherein,
   determining (150) the shape of the identified vessel cross section comprises fitting a general ellipse equation to the identified vessel cross section,
   **characterised in that**
   fitting the general ellipse equation to the identified vessel cross section comprises applying an image momentum method to the ultrasound data, generating a binary mask of the ultrasound data optimized for a set of imaging parameters, and wherein the determining of the shape of the identified vessel cross section is based on the binary mask.

2. The method (100) as claimed in claim 1, wherein deriving (190) the parameter relating to flow comprises determining a flow velocity.

3. The method (100) as claimed in any of claims 1 to 2, wherein the method further comprises guiding a user to obtain ultrasound data from a Doppler angle ($\alpha$) between 10 and 50 degrees, for example between 15 and 40 degrees.

4. The method (100) as claimed in any of claims 1 to 3, wherein the method further comprises:

   generating a beam steering angle based on the vessel axis; and
   adjusting the position of the imaging plane based on the beam steering angle.

5. The method (100) as claimed in any of claims 1 to 4, wherein the method further comprises applying a dynamic gating to the Doppler ultrasound data.

6. The method (100) as claimed in any of claims 1 to 5, wherein the determining (150) the shape of the identified vessel cross section comprises identifying a vessel diameter.

7. The method (100) as claimed in claim 6, wherein the method further comprises:

monitoring a change in vessel diameter over time; and
determining a measure of vessel distensibility based on the change in vessel diameter over time.

8. The method (100) as claimed in any of claims 6 to 7, wherein the parameter relating to flow comprises one or more of:

a flow rate; and
a flow distribution.

9. The method (100) as claimed in any of claims 1 to 8, wherein the ultrasound data comprises B-mode data.

10. A computer program comprising computer program code means which is adapted, when said computer program is run on a computer, to implement the method of any of claims 1 to 9.

11. A medical system adapted to derive a parameter relating to flow from ultrasound data of a vessel, the system comprising:

a processor (22'), wherein the processor is adapted to:

obtain ultrasound data from an imaging plane, wherein the ultrasound data comprises Doppler ultrasound data;
identify a vessel cross section within the imaging plane based on the ultrasound data;
determine a shape of the identified vessel cross section;
determine a vessel axis based on the shape of the identified vessel cross section, wherein the vessel axis extends along the length of the vessel;

determine a Doppler angle between the vessel axis and the imaging plane; and
derive the parameter relating to flow based on the Doppler angle, the vessel axis and the Doppler ultrasound data, and wherein the processor is adapted to determine the shape of the identified vessel cross section by fitting a general ellipse equation to the identified vessel cross section,
**characterised in that**
when fitting the general ellipse equation to the identified vessel cross section the processor is adapted to apply an image momentum method to the ultrasound data, comprising generating a binary mask of the ultrasound data optimized for a set of imaging parameters, and wherein the determining of the shape of the identified vessel cross section is based on the binary mask.

12. The system as claimed in claim 11, wherein the system further comprises an ultrasound transducer in communication with the processor, wherein the ultrasound transducer is adapted to acquire ultrasound data from an imaging plane.

13. The system as claimed in claim 12, wherein the processor is arranged to control the ultrasound transducer to generate a beam steering angle based on the vessel axis; and adjust the position of the imaging plane based on the beam steering angle.

**Patentansprüche**

1. Verfahren (100) zum Erhalten eines Parameters im Zusammenhang mit dem Fluss in einem Gefäß, wobei das Verfahren Folgendes umfasst:

Erhalten (110) von Ultraschalldaten aus einer Bildgebungsebene (120), wobei die Ultraschalldaten Doppler-Ultraschalldaten umfassen;
Identifizieren (130) eines Gefäßquerschnitts (140) innerhalb der Bildgebungsebene basierend auf den Ultraschalldaten;
Bestimmen (150) einer Form des identifizierten Gefäßquerschnitts;
Bestimmen (160) einer Gefäßachse (170) basierend auf der Form des identifizierten Gefäßquerschnitts basierend auf der Annahme eines kreisförmigen Querschnitts in einer Ebene senkrecht zur Gefäßachse, wobei sich die Gefäßachse entlang der Länge des Gefäßes erstreckt;

Bestimmen (180) eines Doppler-Winkels (α) zwischen der Gefäßachse und der Bildgebungsebene; und Ableiten (190) des flussbezogenen Parameters basierend auf dem Doppler-Winkel, der Gefäßachse und den Doppler-Ultraschalldaten, und wobei,

das Bestimmen (150) der Form des identifizierten Gefäßquerschnitts das Anpassen einer allgemeinen Ellipsengleichung an den identifizierten Gefäßquerschnitt umfasst,

**dadurch gekennzeichnet, dass**

das Anpassen der allgemeinen Ellipsengleichung an den identifizierten Gefäßquerschnitt umfasst die Anwendung einer Bildimpulsmethode auf die Ultraschalldaten, das Erzeugen einer binären Maske der Ultraschalldaten, die für einen Satz von Bildgebungsparametern optimiert ist, und wobei das Bestimmen der Form des identifizierten Gefäßquerschnitts auf der binären Maske basiert.

2. Verfahren (100) nach Anspruch 1, wobei das Ableiten (190) des flussbezogenen Parameters das Bestimmen einer Strömungsgeschwindigkeit umfasst.

3. Verfahren (100) nach einem der Ansprüche 1 bis 2, wobei das Verfahren weiterhin das Anleiten eines Benutzers umfasst, um Ultraschalldaten aus einem Doppler-Winkel (α) zwischen 10 und 50 Grad, beispielsweise zwischen 15 und 40 Grad, zu erhalten.

4. Verfahren (100) nach einem der Ansprüche 1 bis 3, wobei das Verfahren weiterhin umfasst:

Erzeugen eines Strahllenkungswinkels basierend auf der Gefäßachse; und
Anpassen der Position der Bildgebungsebene basierend auf dem Strahllenkungswinkel.

5. Verfahren (100) nach einem der Ansprüche 1 bis 4, wobei das Verfahren ferner das Anwenden eines dynamischen Gatings auf die Doppler-Ultraschalldaten umfasst.

6. Verfahren (100) nach einem der Ansprüche 1 bis 5, wobei das Bestimmen (150) der Form des identifizierten Gefäßquerschnitts das Identifizieren eines Gefäßdurchmessers umfasst.

7. Verfahren (100) nach Anspruch 6, wobei das Verfahren weiterhin umfasst:

Überwachung einer Änderung des Gefäßdurchmessers im Laufe der Zeit; und
Bestimmen eines Maßes für die Gefäßdehnbarkeit basierend auf der Änderung des Gefäßdurchmessers im Laufe der Zeit.

8. Verfahren (100) nach einem der Ansprüche 6 bis 7, wobei der flussbezogene Parameter einen oder mehrere der folgenden umfasst:

eine Durchflussrate; und
eine Strömungsverteilung.

9. Verfahren (100) nach einem der Ansprüche 1 bis 8, wobei die Ultraschalldaten B-Mode-Daten umfassen.

10. Computerprogramm, das Computerprogrammcodemittel umfasst, die geeignet sind, das Verfahren nach einem der Ansprüche 1 bis 9 zu implementieren, wenn das Computerprogramm auf einem Computer ausgeführt wird.

11. Medizinisches System, das ausgelegt ist, um aus Ultraschalldaten eines Gefäßes einen flussbezogenen Parameter abzuleiten, wobei das System umfasst:

einen Prozessor (22'), wobei der Prozessor ausgelegt ist zum:

Erhalten von Ultraschalldaten von einer Bildgebungsebene, wobei die Ultraschalldaten Doppler-Ultraschalldaten umfassen;
Identifizieren eines Gefäßquerschnitts innerhalb der Bildgebungsebene basierend auf den Ultraschalldaten;
Bestimmen einer Form des identifizierten Gefäßquerschnitts;
Bestimmen einer Gefäßachse basierend auf der Form des identifizierten Gefäßquerschnitts, wobei sich die Gefäßachse entlang der Länge des Gefäßes erstreckt;

Bestimmen eines Doppler-Winkels zwischen der Gefäßachse und der Bildgebungsebene; und

Ableiten des flussbezogenen Parameters basierend auf dem Doppler-Winkel, der Gefäßachse und den Doppler-Ultraschalldaten, und wobei der Prozessor angepasst ist, um die Form des identifizierten Gefäßquerschnitts durch Anpassen einer allgemeinen Ellipsengleichung an den identifizierten Gefäßquerschnitt zu bestimmen,

**dadurch gekennzeichnet, dass**

der Prozessor beim Anpassen der allgemeinen Ellipsengleichung an den identifizierten Gefäßquerschnitt ausgelegt ist, um eine Bildimpulsmethode auf die Ultraschalldaten anzuwenden, die das Erzeugen einer binären Maske der Ultraschalldaten umfasst, die für einen Satz von Bildgebungsparametern optimiert ist, und wobei die Bestimmung der Form des identifizierten Gefäßquerschnitts auf der binären Maske basiert.

12. System nach Anspruch 11, wobei das System außerdem einen Ultraschallwandler in Kommunikation mit dem Prozessor umfasst, wobei der Ultraschallwandler dazu geeignet ist, Ultraschalldaten von einer Bildgebungsebene zu erfassen.

13. System nach Anspruch 12, wobei der Prozessor so angeordnet ist, dass er den Ultraschallwandler steuert, um einen Strahllenkungswinkel basierend auf der Gefäßachse zu erzeugen; und die Position der Bildebene basierend auf dem Strahllenkungswinkel anzupassen.

**Revendications**

1. Procédé (100) pour obtenir un paramètre relatif au flux dans un vaisseau, le procédé consistant à:

obtenir (110) des données ultrasonores d'un plan d'imagerie (120), où les données ultrasonores comprennent des données ultrasonores Doppler;

identifier (130) une section transversale de vaisseau (140) dans le plan d'imagerie sur la base des données ultrasonores;

déterminer (150) une forme de la section transversale de vaisseau identifiée;

déterminer (160) un axe de vaisseau (170) sur la base de la forme de la section transversale de vaisseau identifiée sur la base d'une hypothèse d'une section transversale circulaire sur un plan perpendiculaire à l'axe du vaisseau, où l'axe du vaisseau s'étend le long de la longueur du vaisseau;

déterminer (180) un angle Doppler ($\alpha$) entre l'axe du vaisseau et le plan d'imagerie; et

dériver (190) le paramètre relatif au flux sur la base de l'angle Doppler, de l'axe du vaisseau et des données ultrasonores Doppler, et où,

la détermination (150) de la forme de la section transversale du vaisseau identifiée comprend l'ajustement d'une équation d'ellipse générale à la section transversale de vaisseau identifiée, **caractérisé en ce que**

l'ajustement de l'équation d'ellipse générale à la section transversale de vaisseau identifiée comprend l'application d'un procédé à impulsion d'image aux données ultrasonores, générant un masque binaire des données ultrasonores optimisées pour un ensemble de paramètres d'imagerie, et où la détermination de la forme de la section transversale de vaisseau identifiée est basée sur le masque binaire.

2. Procédé (100) tel que revendiqué dans la revendication 1, dans lequel la dérivation (190) du paramètre relatif au flux comprend la détermination d'une vitesse de flux.

3. Procédé (100) tel que revendiqué dans l'une quelconque des revendications 1 à 2, dans lequel le procédé consiste en outre à guider un utilisateur pour obtenir des données ultrasonores d'un angle Doppler ($\alpha$) compris entre 10 et 50 degrés, par exemple entre 15 et 40 degrés.

4. Procédé (100) tel que revendiqué dans l'une quelconque des revendications 1 à 3, dans lequel le procédé consiste en outre à:

générer un angle de direction de faisceau sur la base de l'axe du vaisseau; et

ajuster la position du plan d'imagerie sur la base de l'angle de direction du faisceau.

5. Procédé (100) tel que revendiqué dans l'une quelconque des revendications 1 à 4, dans lequel le procédé comprend en outre l'application de fenêtres de contrôle dynamiques aux données ultrasonores Doppler.

6. Procédé (100) tel que revendiqué dans l'une quelconque des revendications 1 à 5, dans lequel la détermination (150) de la forme de la section transversale de vaisseau identifiée comprend l'identification d'un diamètre de vaisseau.

7. Procédé (100) tel que revendiqué dans la revendication 6, dans lequel le procédé consiste en outre à:

surveiller un changement du diamètre du vaisseau au fil du temps; et
déterminer une mesure de distensibilité du vaisseau sur la base du changement du diamètre du vaisseau au fil du temps.

8. Procédé (100) tel que revendiqué dans l'une quelconque des revendications 6 à 7, dans lequel le paramètre relatif au flux comprend un ou plusieurs entre:

un débit; et
une distribution de flux.

9. Procédé (100) tel que revendiqué dans l'une quelconque des revendications 1 à 8, dans lequel les données ultrasonores comprennent des données en mode B.

10. Programme informatique comprenant des moyens de code de programme informatique qui sont adaptés, lorsque ledit programme informatique est exécuté sur un ordinateur, à mettre en œuvre le procédé selon l'une quelconque des revendications 1 à 9.

11. Système médical adapté pour dériver un paramètre relatif au flux à partir de données ultrasonores d'un vaisseau, le système comprenant:

un processeur (22'), où le processeur est adapté pour :

obtenir des données ultrasonores d'un plan d'imagerie, où les données ultrasonores comprennent des données ultrasonores Doppler;
identifier une section transversale de vaisseau dans le plan d'imagerie sur la base des données ultrasonores;
déterminer une forme de la section transversale de vaisseau identifiée;
déterminer un axe de vaisseau sur la base de la forme de la section transversale de vaisseau identifiée, où l'axe du vaisseau s'étend le long de la longueur du vaisseau;
déterminer un angle Doppler entre l'axe du vaisseau et le plan d'imagerie; et
dériver le paramètre relatif au flux sur la base de l'angle Doppler, de l'axe du vaisseau et des données ultrasonores Doppler, et où le processeur est adapté pour déterminer la forme de la section transversale de vaisseau identifiée en ajustant une équation d'ellipse générale à la section transversale de vaisseau identifiée,
**caractérisé en ce que**

lors de l'ajustement de l'équation d'ellipse générale à la section transversale de vaisseau identifiée, le processeur est adapté pour appliquer un procédé d'impulsion d'image aux données ultrasonores, comprenant la génération d'un masque binaire des données ultrasonores optimisé pour un ensemble de paramètres d'imagerie,
et où la détermination de la forme de la section transversale de vaisseau identifiée est basée sur le masque binaire.

12. Système selon la revendication 11, dans lequel le système comprend en outre un transducteur à ultrasons en communication avec le processeur, dans lequel le transducteur à ultrasons est adapté pour acquérir des données ultrasonores d'un plan d'imagerie.

13. Système selon la revendication 12, dans lequel le processeur est agencé pour commander le transducteur à ultrasons pour générer un angle de direction de faisceau sur la base de l'axe du vaisseau; et pour ajuster la position du plan d'imagerie sur la base de l'angle de direction du faisceau.

FIG. 1

FIG. 2

FIG. 3a

200

120

140

215

225

FIG. 3b

FIG. 4

FIG. 5

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120078106 A
- US 6663568 B
- US 5997479 A, Savord
- US 6013032 A, Savord
- US 6623432 B, Powers
- US 6283919 B, Roundhill
- US 6458083 B, Jago
- US 6443896 B, Detmer
- US 6530885 B, Entrekin

**Non-patent literature cited in the description**

- **CHAN, T.F. ; SANDBERG Y. B.** Active contours without edges for Vector-valued Image. *Journal of Visual Communication and Image Representation,* 2000, vol. 11, 130-141
- **CHAN, T. F. ; VESE, L. A.** Active contours without edges. *IEEE Transactions on Image Processing,* 2001, vol. 10 (2), 266-277
- **CHAN, T. F. ; VESE, L. A.** A Multiphase level set framework for image segmentation using the Mumford and Shah model. *International Journal of Computer Vision,* 2002, vol. 50 (3), 271-293
- **SOBEL, I. ; FELDMAN, G.** A 3x3 Isotropic Gradient Operator for Image Processing. *Stanford Artificial Intelligence Project (SAIL),* 1968
- **CANNY, J.** A Computational Approach To Edge Detection. *IEEE Trans. Pattern Analysis and Machine Intelligence,* 1986, vol. 8 (6), 679-698